(19) Europäisches Patentamt

European Patent Office

Office européen des brevets

(11) Publication number: **0 287 204**
**A2**

(12) **EUROPEAN PATENT APPLICATION**

(21) Application number: 88301620.6

(22) Date of filing: **25.02.88**

(51) Int. Cl.4: **A61K 31/635 , A61K 33/38**

The title of the invention has been amended (Guidelines for Examination in the EPO, A-III, 7.3).

(30) Priority: **25.02.87 US 18624**

(43) Date of publication of application:
**19.10.88 Bulletin 88/42**

(84) Designated Contracting States:
**AT BE CH DE FR GB IT LI NL SE**

(71) Applicant: **The Trustees of Columbia University in the City of New York Morningside Heights New York, NY 10027(US)**

(72) Inventor: **Fox, Charles L., Jr. 290 West End Avenue - Apt. 4C New York, NY 10023(US)**

(74) Representative: **Ford, Michael Frederick et al MEWBURN ELLIS & CO. 2/3 Cursitor Street London EC4A 1BQ(GB)**

(54) **Silver sulfadiazine useful for inhibiting the transmission of the aids virus.**

(57) A method of inhibiting the transmission of AIDS virus in humans upon sexual intercourse comprises topically applying an effective antiviral amount of silver sulfadiazine to a sexual canal, such as a vaginal canal or an anal canal, of a human prior to or during sexual intercourse.

EP 0 287 204 A2

# METHOD OF INHIBITING THE TRANSMISSION OF AIDS VIRUS

## BACKGROUND OF THE INVENTION

The present invention relates to a method of inhibiting the transmission of AIDS virus.

AIDS, i.e., acquired immune deficiency syndrome, is a fatal catastrophic disease infecting millions of people worldwide, particularly in central Africa but also in the United States. AIDS is caused by the AIDS virus.

A controversy exists in the scientific community in regard to the proper nomenclature for the AIDS virus. Thus in the scientific literature the AIDS virus is variously designated as HIV (human immunodeficiency virus), LAV (lumphadenopathy - associated virus), ARV (AIDS -related virus) and HTLV-III (human T-cell leukemia virus-III). The AIDS virus is a retrovirus, which means that its genetic material is ribonucleic acid (RNA) rather than deoxyribonucleic acid (DNA). Retroviruses carry with them an enzyme called reverse transcriptase, which catalyzes transcription of viral RNA into double-helical DNA that then integrates into the genome of the infected cell where it is known as a provirus. Transcription of this provirus produces new viral RNA and proteins.

AIDS can be transmitted to a person by transfusing that person with blood containing the AIDS virus. AIDS can also be transmitted to a person by homosexual or heterosexual intercourse with a partner infected with the AIDS virus. Transmission of the AIDS virus is facilitated by preexisting forms of venereal disease, such as gonorrhea. Scientists suspect that the AIDS virus is spread easily in sexual intercourse, which often involves tearing of tissue that would abet the entry of the AIDS virus into the bloodstream.

Heretofore, condoms have been used in an attempt to prevent the transmission of AIDS virus upon sexual intercourse. However, the improper use of condoms or the perforation thereof during sexual intercourse renders them only partially effective. Accordingly, there is a pressing need for a better method of inhibiting the transmission of AIDS virus in humans upon sexual intercourse.

## SUMMARY OF THE INVENTION

Accordingly, it is a principal object of the present invention to provide an inexpensive, easily available and convenient method of inhibiting the transmission of AIDS virus in humans upon sexual intercourse.

The Charles L. Fox, Jr., U.S. patent No. 3,761,590 discloses that silver sulfadiazine is an antibacterial agent useful in treating burns in man and animal. Silver sulfadiazine is also known to be effective against certain viruses, such as herpes simplex and herpes zoster. Silver sulfadiazine is further known to be effective against treponema pallidum and gonorrhea and is spermicidal.

It has now been found quite unexpectedly that silver sulfadiazine is an effective antiviral agent against the AIDS virus. Accordingly, the method of the present invention of inhibiting the transmission of AIDS virus in humans upon sexual intercourse comprises topically applying an effective antiviral amount of silver sulfadiazine to a sexual canal of a human prior to or during sexual intercourse. The known effectiveness of silver sulfadiazine against gonorrhea assists in its effectiveness in inhibiting the transmission of AIDS virus.

## DETAILED DESCRIPTION

As noted above, the method of the present invention of inhibiting the transmission of AIDS virus in humans upon sexual intercourse comprises topically applying an effective antiviral amount of silver sulfadiazine to a sexual canal of a human prior to or during sexual intercourse. The sexual canal can be a vaginal canal or an anal canal.

The silver sulfadiazine can be applied in various ways, notably as (a) a dispersion in a water-dispersible, hydrophilic carrier; (b) as a dispersion in a substantially water-insoluble hydrophobic carrier; (c) as a dispersion in a semi-soft or cream-like, water-dispersible or water-soluble oil-in-water emulsion carrier; or (d) as a dispersion in an aqueous sucrose solution carrier, e.g., an approximately 25%-50% by weight aqueous sucrose solution. The carrier contains an amount in a range of from about 0.1 to about 10% by weight of silver sulfadiazine dispersed therein.

Dispersions of silver sulfadiazine in a water-dispersible hydrophilic carrier or in a substantially water-insoluble hydrophobic carrier or in a semi-soft or cream-like, water-dispersible or water-soluble oil-in-water emulsion carrier suitable for use in the method of the present invention are disclosed in the above-mentioned Charles L. Fox, Jr., U.S. patent No. 3,761,590, the entire disclosure of which patent is hereby incorporated by reference herein.

The silver sulfadiazine/carrier composition or dispersion useful in the method of the present invention can be contained in a squeezable tube having an applicator nozzle. The silver sulfadiazine-containing dispersion can be topically applied to a sexual canal of a human prior to sexual intercourse by inserting the nozzle of the tube into the sexual canal and squeezing the tube to force the contents thereof into the sexual canal.

Alternatively, the silver sulfadiazine can be topically applied to a sexual canal of a human during sexual intercourse by coating the penis itself or coating the condom for sheathing the penis with a lubricant material, such as K-Y Jelly (Johnson & Johnson), containing the silver sulfadiazine as a component thereof. The condom can also either be coated or impregnated with silver sulfadiazine by soaking the condom material in a coating or impregnating base material containing the silver sulfadiazine and thereafter drying the soaked condom material.

The following in vitro test results, which are highly predictive of and correlate with in vivo efficacy, establish the unexpected finding that silver sulfadiazine is an effective antiviral agent against the AIDS virus and hence can be used to inhibit the transmission of AIDS virus in humans upon sexual intercourse by topically applying an effective antiviral amount of silver sulfadiazine to a sexual canal of a human prior to or during sexual intercourse.

IN VITRO TESTS

Materials:

1. Silver sulfadiazine (AgSD) - 1% by weight in 50% by weight aqueous sucrose solution and 0.5% by weight in 25% by weight aqueous sucrose solution.

2. HTLV-III (Stock 21) - 10,000 fold concentrate (Bionetics Research) diluted 1:10 in medium. est. titer $10^7$-$10^8$/ml (Actual titer 105.5/ml) Stock diluted 1:10 before use.

3. Target cells - H9/Gallo

Rationale of Study Design:

Due to the relatively low titers achievable with the AIDS virus (HTLV-III), it was necessary to devise means for separating the bulk of the AgSD from the AIDS virus to be assayed. After a number of preliminary experiments it was found that the best method of those investigated was to rapidly pass the AgSD/AIDS virus mixture over a Sephadex G-25M column and recovering the AIDS virus containing void volume, followed by a polyethylene glycol (PEG) precipitation of the AIDS virus to further remove the AgSD.

To determine recovery of the AIDS virus after use of these steps a control virus preparation was similarly processed.

To determine whether the procedure removed all of the AgSD "Stop controls" were carried out, i.e., AgSD was processed by the above procedure, and then the AIDS virus was added to the final PEG precipitate. If the titer of this is similar to the control preparation, it may be concluded that little or no AgSD was present in the PEG precipitate. In this case, it is justified to conclude that any AIDS virus kill occurred within the 10 minutes prior to separation. If AIDS virus kill is observed in the stop control, this indicates inadequate separation; thus in this case the observed inactivation may not have taken place only during the initial 10 minute contact time. However, the failure of the separation process to remove all virucidal activity (AgSD) indicates the latter to be very strong, thus it is likely that the rate of AIDS virus kill is even greater in these cases.

Experimental Procedure:

1. Quick thaw 4.0 ml of Stock 21 and hold at 0°C.

2. Dilute 0.7 + 6.3 CIM (a culture medium) to make $10^{-2}$ dilution for stop controls.

3. Place AgSD, or CIM in sterile microfuge tubes at room temperature in accordance with the assay mixture chart below.

4. Add 60μl of $10^{-1}$ Stock 21 to indicated tubes (see chart below) in groups of 6 tubes. Hold for 10 min. at room temperature after vortexing to mix.

5. Stop Procedure

A. Columns (Sephadex G25M) are washed with 18 X 4 ml complete medium on previous day to remove all sodium azide using sterile conditions.

B. 0.5 ml of sample is placed on column until it passes fritted disc on column. Immediately 4 ml of medium is placed on column. 3 ml is discarded, then 1 ml is collected into sterile microfuge tubes containing 0.35 ml 30% PEG 6000 in PBS (phosphate buffer solution).

C. Microfuge tubes are held at 0° for at least 30 min. and are then centrifuged for 1 min in the microfuge. Pellets are resuspended in 0.4 ml of CIM or $10^{-2}$ HTLV III in CIM as indicated on the assay mixture chart below. Tubes are mixed by pipette and vortexed.

6. Assay in quadruplicate with 10 fold dilutions in CIM. Add 50μl of 2.4 x $10^6$/ml H9 cells, conditioned in CIM for 1 hr at 37°C., to each 100μl containing culture.
Feed 25μl infection medium on days 4, 7 and 10. Harvest for reverse transcriptase assay on day 14.

7. Check for cytotoxicity by examination of cultures on day 4.

| Sample No. | Material | HTLV-III (Stock 21) $10^{-1}$ | Mixture CIM | AgSD | Stop Procedure | PEG Pellet Resuspended in (0.5ml) | $Log_{10}$ $TCID_{50}$ per/ml [**] | Log Kill [***] | Cytoxicity |
|---|---|---|---|---|---|---|---|---|---|
| 1 | HTLV-III (Stock 21) ($10^{-1}$) | $60\mu l$ | $540\mu l$ | – | – | – | 4.5 | – | 0 |
| 2 | " | " | " | – | Column + PEG | CIM | 4.25 | – | 0 |
| 3 | 1% AgSD in 50% aqueous sucrose solution | " | – | 540 | Cent.[*] " " | CIM | 2.0 | 2.25 | 0 |
| 4 | " | – | | 600 | " " " | $10^{-2}$ HTLV-III (Stop Control) | 3.25 | – | 0 |
| 5 | 0.5% AgSD in 25% aqueous solution | $60\mu l$ | – | 540 | " " " | CIM | 2.25 | 2.0 | 0 |
| 6 | " | – | | 600 | " " " | $10^{2}$ HTLV-III (Stope Control) | 3.75 | – | 0 |

[*] Centrifuge 1 minute in microfuge – place supernatant on column

[**] $TCID_{50}$ = Tissue Culture Infecting Dose$_{50}$

[***] Compared to Sample No. 2

Conclusions:

Silver sulfadiazine (AgSD) killed between 2 and 2.25 $Log_{10}$ $TCID_{50}$ of HTLV-III. This is a definite antiviral effect.

The antiviral efficacy of AgSD against the AIDS virus (HTLV-III) increases as the concentration of AgSD used is increased and as the contact time is increased.

## Claims

1. A method of inhibiting the transmission of AIDS virus in humans upon sexual intercourse which comprises topically applying an effective antiviral amount of silver sulfadiazine to a sexual canal of a human prior to or during sexual intercourse.

2. A method in accordance with claim 1 wherein the sexual canal is a vaginal canal.

3. A method in accordance with claim 1 wherein the sexual canal is an anal canal.

4. A method in accordance with claim 1 wherein said silver sulfadiazine is applied as a dispersion in a water-dispersible, hydrophilic carrier.

5. A method in accordance with claim 1 wherein said silver sulfadiazine is applied as a dispersion in a substantially water-insoluble hydrophobic carrier.

6. A method in accordance with claim 1 wherein said silver sulfadiazine is applied as a dispersion in a semi-soft or cream-like, water-dispersible or water-soluble oil-in-water emulsion carrier.

7. A method in accordance with claim 1 wherein said silver sulfadiazine is applied as a dispersion in an aqueous sucrose solution carrier.

8. A method in accordance with claims 4, 5, 6 or 7, wherein said carrier contains an amount in the range from about 0.1 to about 10 percent by weight of silver sulfadiazine dispersed therein.

9. A method in accordance with claim 1 wherein said silver sulfadiazine is applied as a component of a lubricant material coating a penis.

10. A method in accordance with claim 1 wherein said silver sulfadiazine is applied as a component of a lubricant material coating a condom for sheathing a penis.

11. A method in accordance with claim 1 wherein said silver sulfadiazine is applied as a component of a coating on a condom for sheathing a penis.

12. A method in accordance with claim 1 wherein said silver sulfadiazine is applied as a component of an impregnant in a condom for sheathing a penis.

13. Use of silver sulfadiazine for the manufacture of a product for topical application to the human body to inhibit the transmission of AIDS virus.

14. A composition for inhibiting the transmission of AIDS virus in humans upon sexual intercourse which comprises an effective anti-viral amount of silver sulfadiazine.